# EUROPÄISCHE PATENTANMELDUNG

(11) **EP 0 956 845 A2**
(43) Veröffentlichungstag der Anmeldung: **17.11.1999**
(21) Anmeldenummer: 99107865.0
(22) Anmeldetag: 21.04.1999
(51) Int. Cl.: A61F 15/00

(54) **Aufnahme- und Entnahmevorrichtung für Wattestäbchen**

(30) Priorität: 29.04.1998 DE 29807696 U
(71) Anmelder: Schmitt, Rolf-Peter, 56424 Staudt (DE)
(72) Erfinder: Schmitt, Rolf-Peter, 56424 Staudt (DE)
(74) Vertreter: Kossobutzki, Walter, Dipl.-Ing.(FH)

(57) **Zusammenfassung**

Die Erfindung bezieht sich auf eine Vorrichtung zur Aufnahme- und Entnahme von Wattestäbchen (14) und dergleichen, bestehend aus einem die Wattestäbchen (14) aufnehmenden Gehäuse (2).

Um eine gute Aufnahme der Wattestäbchen (14) zu gewährleisten und immer nur ein Wattestäbchen (14) ohne Probleme entnehmen zu können, besitzt das Gehäuse (2) mindestens zwei parallel hintereinander verlaufende, mehrere übereinanderliegende Wattestäbchen (14) mit Spiel aufnehmende Schächte (12) mit geneigtem Boden (2d), wobei der vorderste Schacht (12) in einen nur ein Wattestäbchen (14) aufnehmenden Schieber (15) übergeht, unter dem eine Entnahmekammer (10) angeordnet ist und eine zwischen zwei aufeinanderfolgenden Schächten (12) befindliche Trennwand (11) ist bedarfsweise um ein vorgegebenes Maß anhebbar.

## Beschreibung

Die Erfindung bezieht sich auf eine Vorrichtung zur Aufnahme und Entnahme von Wattestäbchen oder dergleichen, bestehend aus einem die Wattestäbchen aufnehmenden Gehäuse.

Wattestäbchen werden in besonderen Verpackungen, vielfach in Dosen mit Deckeln, angeboten. Nach dem Kauf werden diese Verpackungen meist in Schränken von Badezimmern untergebracht und aus denselben - je nach Bedarf - die Wattestäbchen für den Gebrauch entnommen. Dabei ist es, in Abhängigkeit von der Art der Verpackung, äußerst umständlich, die Wattestäbchen von Hand aus der Verpackung zu entnehmen. Meistens wird mehr als ein Wattestäbchen erfaßt oder an einem Wattestäbchen haftet ein weiteres Wattestäbchen, welches dann wieder in die Verpackung zurückfällt und dort für Unordnung sorgt und damit die Entnahme eines weiteren Wattestäbchens behindert. Darüber hinaus wird es auch vielfach als zeitraubend angesehen, daß - wenn rasch ein Wattestäbchen benötigt wird - zuerst ein Schrank und eine Verpackung geöffnet werden müssen, die dann auch wieder zu schließen sind.

Der Erfindung liegt deshalb die Aufgabe zugrunde, eine Vorrichtung zur Aufnahme und Entnahme von Wattestäbchen oder dergleichen zu schaffen, die eine gute Aufnahme der Wattestäbchen gewährleistet und mit der, ohne daß irgendwelche Probleme auftreten, sichergestellt ist, daß jeweils immer nur ein Wattestäbchen entnommen werden kann.

Zur Lösung dieser Aufgabe wird gemäß der Erfindung bei einer Vorrichtung der eingangs beschriebenen Gattung vorgeschlagen, daß das Gehäuse mindestens zwei parallel hintereinander verlaufende, mehrere übereinanderliegende Wattestäbchen mit Spiel aufnehmende Schächte mit geneigtem Boden besitzt, daß der vorderste Schacht in einen nur ein Wattestäbchen aufnehmenden Schieber übergeht, unter dem eine Entnahmekammer angeordnet ist, und daß eine zwischen zwei aufeinanderfolgenden Schächten befindliche Trennwand bedarfsweise um ein vorgegebenes Maß anhebbar ist.

Bei einer derartig ausgebildeten Vorrichtung werden die Wattestäbchen zunächst einzeln in die Schächte eingefüllt. Durch entsprechendes Bewegen des Schiebers wird jeweils nur ein Wattestäbchen an die Entnahmekammer übergeben, aus der es dann leicht erfaßt und herausgenommen werden kann. Das bisher übliche Suchen nach Wattestäbchen entfällt. Die erfindungsgemäße Vorrichtung kann in einfacher Weise, beispielsweise in einem Bad, an einer Wand befestigt werden, wobei es zweckmäßig ist, diese Vorrichtung bzw. deren Gehäuse mit anderen Gehäusen, die Zahncreme, Zahnbürsten, Papiertaschentücher oder Wattepads aufnehmen, zu integrieren wird, so daß eine große Baueinheit entsteht, die die in einem Bad benötigten Dinge stets griffbereit hält.

Weitere Merkmale einer Vorrichtung gemäß der Erfindung sind in den Ansprüchen 2 bis 6 offenbart.

Die Erfindung wird nachfolgend anhand eines in einer Zeichnung in stark vereinfachter Weise dargestellten Ausführungsbeispieles näher erläutert.

In dieser Zeichnung ist eine Vorrichtung 1 zur Aufnahme und Entnahme von Wattestäbchen 14 in vereinfachter Weise dargestellt, die zunächst aus einem Gehäuse 2, beispielsweise aus durchscheinendem Kunststoff, mit rechteckigem Querschnitt besitzt. Das Gehäuse 2 weist eine Vorderwand 2a, eine Rückwand 2b und zwei Seitenwände 2c auf. Von der Rückwand 2b führt ein beispielsweise unter einem Winkel von 45° nach unten verlaufender Boden 2d bis etwa zur Innenfläche der Vorderwand 2a. In dem unteren Bereich gehen sowohl die Seitenwände 2c als auch der Boden 2d in eine Grundplatte 3 über. Im dargestellten Ausführungsbeispiel ist in diese Grundplatte 3 eine Aussparung 4 eingearbeitet, in die ein Schieber 5 eingesetzt ist. Dieser Schieber 5 steht normalerweise unter der Spannung von mindestens einer vorgespannten Zugfeder 6. In die gezeichnete Lage des Schiebers 5 kann derselbe nur entgegen der Kraft der Feder 6 verschoben werden.

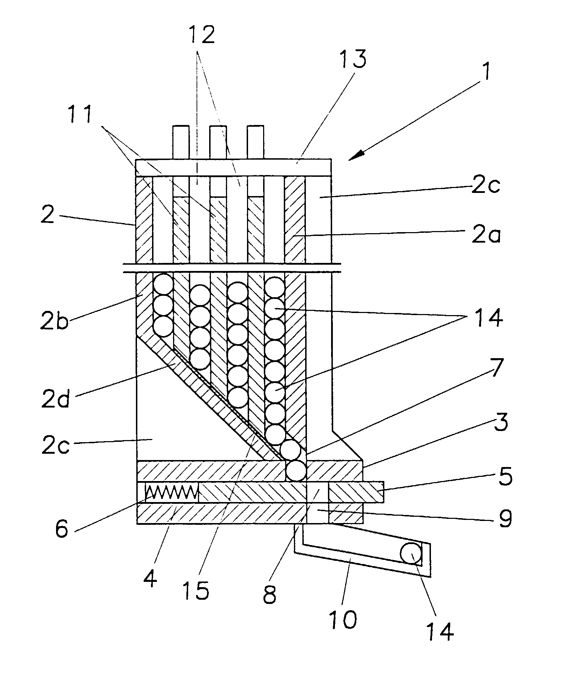

In dem Bereich zwischen Boden 2d, Vorderwand 2a und Grundplatte 3 ist ein Kanal 7 eingearbeitet, der in der Grundstellung des Schiebers 5 in eine Aussparung 8 übergeht. In der in der Zeichnung dargestellten Lage des Schiebers 5 befindet sich die Aussparung 8 über einer Fallöffnung 9, die in der Grundplatte 3 eingearbeitet ist. Unter der Grundplatte 3 und damit unterhalb der Fallöffnung 9 befindet sich eine Entnahmekammer 10.

Der Abstand zwischen den beiden Seitenwänden 2c des Gehäuses 2 ist so groß ausgebildet, daß er geringfügig größer ist als die längsten, auf dem Markt befindlichen Wattestäbchen 14. In die Seitenwände 2c sind nun an der Innenseite senkrechte Nuten eingearbeitet, in die Trennwände 11 von oben eingeschoben werden können. Durch diese Trennwände 11 werden zwischen denselben und der Vorderwand 2a und der Rückwand 2b Schächte 12 gebildet, deren Breite geringfügig größer als der größte Durchmesser von Wattestäbchen ist. Beispielsweise durch Entfernen eines Deckels 13 können nun die Schächte 12, wie teilweise angedeutet, mit den Wattestäbchen 14 gefüllt werden.

In der dargestellten Lage der Trennwände 11 fallen die im vordersten Schacht 12 befindlichen bzw. eingefüllten Wattestäbchen 14 zunächst in den Kanal 7 und von dort - in der Grundstellung des Schiebers 5 - in die Aussparung 8. Dabei bleibt das unterste Wattestäbchen 14 an der Unterseite der Aussparung 4 im Schieber 5 liegen. Wird nun der Schieber 5, in dessen Aussparung 8 sich das Wattestäbchen 14 befindet, nach vorne gezogen, fällt dasselbe nach unten in die Entnahmekammer 10, aus der es dann, wie bereits erwähnt, herausgenommen werden kann.

Alle Trennwände 11 sind an ihrer Unterseite mit einer Leitplatte 15 bestückt, die parallel zum Boden 2c verläuft und die sich auch unterhalb des Querschnittes eines Schachtes 12 erstreckt. Wurden nun aus dem vordersten Schacht 12 alle Wattestäbchen 14 entnommen, wird die erste Trennwand 11 zusammen mit ihrer Leitplatte 15 nach oben bewegt. Dadurch wird der Kanal 7 nach hinten und oben verlängert, so daß jetzt die Wattestäbchen 14 aus dem zweiten Schacht 12 in den Kanal 7 und von dort über die Grundplatte 3 zur Aussparung 8 des Schiebers 5 gelangen. Ist auch dieser Schacht 12 leer, wird die nächste Trennwand 11 ebenfalls um einen vorgegebenen Betrag nach oben gezogen, so daß jetzt der nächste Schacht 12 für die Abgabe von Wattestäbchen 14 bereit ist. Sind alle Schächte 12 leer, werden dieselben erneut wieder mit Wattestäbchen gefüllt.

In Abänderung des erläuterten Ausführungsbeispieles ist es möglich, in dem Deckel 13 Öffnungen vorzusehen, über die die Schächte 12 mit Wattestäbchen 14 gefüllt werden können.

## Patentansprüche

1. Vorrichtung zur Aufnahme und Entnahme von Wattestäbchen oder dergleichen, bestehend aus einem die Wattestäbchen aufnehmenden Gehäuse,
dadurch gekennzeichnet,
daß das Gehäuse (2) mindestens zwei parallel hintereinander verlaufende, mehrere übereinanderliegende Wattestäbchen (14) mit Spiel aufnehmende Schächte (12) mit geneigtem Boden (2d) besitzt, daß der vorderste Schacht (12) in einen nur ein Wattestäbchen (14) aufnehmenden Schieber (15) übergeht, unter dem eine Entnahmekammer (10) angeordnet ist, und daß eine zwischen zwei aufeinanderfolgenden Schächten (12) befindliche Trennwand (11) bedarfsweise um ein vorgegebenes Maß anhebbar ist.

2. Vorrichtung nach Anspruch 1,
dadurch gekennzeichnet,
daß der Schieber (5) durch eine vorgespannte Feder (6) in seiner Grundstellung gehalten wird.

3. Vorrichtung nach Anspruch 1,
dadurch gekennzeichnet,
daß dem Schieber (5) ein Griff zugeordnet ist.

4. Vorrichtung nach mindestens einem der Ansprüche 1 bis 3,
dadurch gekennzeichnet,
daß jede Trennwand (11) an ihrem unteren Ende mit einer der Neigung des Bodens (2d) entsprechenden Leitplatte (15) versehen ist, die sich bis zur vorhergehenden Wand bzw. Trennwand (2a, 11) erstreckt.

5. Vorrichtung nach mindestens einem der Ansprüche 1 bis 4,
dadurch gekennzeichnet,
daß das Gehäuse (2) einen mit Einfüllöffnungen versehen Deckel (13) aufweist.

6. Vorrichtung nach mindestens einem der Ansprüche 1 bis 4,
dadurch gekennzeichnet,
daß das Gehäuse (2) einen abnehmbaren Deckel (13) besitzt.
